(19) Europäisches Patentamt
European Patent Office
Office européen des brevets

(11) Veröffentlichungsnummer: **0 190 322**
**B1**

(12) # EUROPÄISCHE PATENTSCHRIFT

(45) Veröffentlichungstag der Patentschrift: **24.10.90**

(21) Anmeldenummer: **85904246.7**

(22) Anmeldetag: **26.08.85**

(86) Internationale Anmeldenummer:
**PCT/EP85/00434**

(87) Internationale Veröffentlichungsnummer:
**WO 86/01502 13.03.86 Gazette 86/06**

(51) Int. Cl.$^5$: **C 07 C 209/14,**
C 07 C 227/04, C 07 C 213/02,
C 07 H 5/06

(54) **VERFAHREN ZUR HERSTELLUNG VON AMINOVERBINDUNGEN AUS HYDROXYLVERBINDUNGEN.**

(30) Priorität: **28.08.84 DE 3431591**

(43) Veröffentlichungstag der Anmeldung:
**13.08.86 Patentblatt 86/33**

(45) Bekanntmachung des Hinweises auf die Patenterteilung:
**24.10.90 Patentblatt 90/43**

(84) Benannte Vertragsstaaten:
**AT BE CH DE FR GB IT LI LU NL SE**

(56) Entgegenhaltungen:
**CH-A- 521 939**
**DE-A-3 303 344**
**Angewandte Chemie International Edition, Band 22, Nr. 1, 1983, Verlag Chemie GMBH, Weinheim (DE) F. EFFENBERG et al.: "Trifluoromethanesulfonates of alpha - hydroxycarboxylates- Educts for the racemization- Free synthesis of N-substituted alpha-amino acids", Seiten 65,66**
**Liebigs Annalen der Chemie, Nr. 6, 1981 R.Kimmich et al.: "C-4-Triflat Substitution durch Azid bei 2,3-Anhydrozuckern; ein neuer Weg zu 4-Amino-4-desoxyzuckern", Seiten 1100-1104, siehe S.1103**

(73) Patentinhaber: **DIAMALT AKTIENGESELLSCHAFT**
**Georg-Reismüller-Strasse 32-36**
**D-8000 München 50 (DE)**

(72) Erfinder: **VOELTER, Wolfgang**
**Panoramastr. 79**
**D-7400 Tübingen (DE)**
Erfinder: **KOWOLLICK, Wolfgang**
**Schwalbenstr. 13**
**D-7403 Ammerbuch 2 (DE)**

(56) Entgegenhaltungen:
**Journal of the Chemical Society, Chemical Communications, Nr.18, '84A.Malik et al.: "Classical Synthesis of a New Class of Compounds via Coupling of Sugars and Amino Acids, Seiten 1229-1231**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 190 322 B1

**Beschreibung**

Die Erfindung betrifft ein allgemein gültiges Verfahren, das auch als Voelter-Reaktion bezeichnet wird, zur Herstellung von primären Aminoverbindungen aus Hydroxylverbindungen.

Aminoverbindungen sind eine der Klassen von organischen Verbindungen, welche wohl am besten untersucht sind. Zu ihrer Herstellung gibt es zahlreiche Verfahren, wie beispielsweise die Reduktion von Nitroverbindungen, die Umsetzung von Halogeniden mit Ammoniak, die reduktive Aminierung, die Reduktion von Nitrilen, den Hoffmann'schen Abbau von Amiden oder die Alkylierung von Aminen. Alle diese Verfahren sind gut bekannt und finden in der Praxis zur technischen Darstellung von Aminoverbindungen vielfach Verwendung.

In manchen Fällen ist die Darstellung von Aminoverbindungen jedoch mit Schwierigkeiten verbunden. Dies gilt beispielsweise dann, wenn in komplizierte Moleküle Aminogruppen eingeführt werden sollen oder wenn die Moleküle noch andere Gruppen enthalten, die bei den durchgeführten Reaktionen ebenfalls reagieren. Die stereospezifische Einführung von primären Aminogruppen ist ebenfalls in vielen Fällen mit Schwierigkeiten verbunden. Dies gilt insbesondere auf dem Gebiet der Steroide, Antibiotika, Hormone und Kohlehydrate, insbesondere der Zucker.

Zuckerderivate, bei denen eine oder mehrere Hydroxylgruppen durch Aminogruppen ersetzt sind, werden als Aminozucker bezeichnet. In Glykoproteinen und Heteroglykanen werden fast ausnahmslos 2-Aminohexosen gefunden. 3-, 4-, 5- und 6-Aminozucker sowie Diaminozucker treten bei verschiedenen Stoffwechselprodukten von Pilzen und Bakterien sowie als Bestandteile natürlicher Antibiotika auf. Manche der Aminozucker sind sogar selbst antibiotisch aktiv. So wirkt beispielsweise Streptozotocin, ein Monosaccharid-Aminoglykosid aus Streptomyces achromogenes, u.a. als Zytostatikum.

Zu dem umfangreichen Spektrum der natürlichen Aminozucker gesellen sich laufend neue synthetische Präparate, darunter auch Tri- und Tetraaminozucker. Die raschen Fortschritte auf diesem Gebiet sind nötig, um mit der Resistenzentwicklung Schritt zu halten. Mit der Zeit bilden Bakterien nämlich Enzyme, die das Molekül durch Acetylierung, Phosphorylierung oder Adenylierung desaktivieren. Man ist daher bemüht, immer neue Varianten von Aminoglykosiden zu entwickeln.

Unter den bisher erfaßten Aminosuckerderivaten findet man solche, die über eine Peptidbindung mit Aminosäuren verknüpft sind. 2-Amino-2-deoxy-gluconsäure wiederum, durch Cannizzaro-Reaktion aus Glucosamin erhältlich, ist zwar eine (D)-α-Aminocarbonsäure, aber nicht mehr als Zucker anzusehen.

Gelingt es jedoch, Zucker und Aminosäuren über den Stickstoff als sekundäres Amin miteinander zu koppeln, dann verfügt man nicht nur über potentielle Antibiotika sondern auch über interessante Ausgangsstufen für weitere Synthesen. Der Zugang zu Glycopeptiden mit der Zuckerkomponente an beliebiger Stelle der Peptidkette stellt nur einen Aspekt dar.

Aus der DE—A—3 303 344 ist die Umsetzung von speziellen Oxycarbonsäuren mit gegebenenfalls substituierten Aminosäuren oder deren Estern zu N-alkylierten Aminosäuren und deren Estern bekannt. In "Angewandte Chemie", International Edition in English (1983), Jahrgang 22, Nr. 1, Seiten 65/66 wird die Umsetzung von Triflatderivaten von α-Hydroxycarbonsäuren oder deren Estern mit primären oder sekundären Aminen, insbesondere mit Aminosäuren zu N-substituierten α-Aminocarbonsäureestern beschrieben. Die Umsetzung ist besonders vorteilhaft für die Synthese von Phenylalanin- und Asparaginsäurederivaten. Bei diesen bekannten Verfahren werden die Triflate, die mit Aminen umgesetzt werden sollen, zuvor durch eine zur $OSO_2CF_3$-Gruppe α-ständige COOR-Gruppe stabilisiert.

Gemäß "Liebigs Annalen der Chemie", 1981, Heft 6, Seite 1101, werden C-4-Triflatderivate von 2,3-Anhydrozuckern mit Natriumazid umgesetzt; dann wird die in den Zucker eingeführte Azidogruppe reduziert, um die entsprechenden 4-Amino-4-desoxyzucker zu erhalten. Es handelt sich hier um eine Umsetzung mit den geladenen, als sehr reaktive Nukleophile bekannten Azidionen.

Der vorliegenden Erfindung liegt die Aufgabe zugrunde, ein einfaches Verfahren zur Herstellung von primären Aminoverbindungen zur Verfügung zu stellen. Bei dem Verfahren sollen leicht zugängliche Ausgangsmaterialien verwendet werden, es soll so ablaufen, daß keine oder kaum Nebenprodukte gebildet werden und die Ausbeuten hoch sind, und das erhaltene Produkt soll leicht aus dem Reaktionsgemisch isoliert werden können. Das Verfahren soll auch mit kompliziert aufgebauten Molekülen, wie Steroiden, Naturstoffen, Antibiotika, Hormonen und Kohlehydraten durchführbar sein und insbesondere die einfache Herstellung von Aminodesoxyzuckerderivaten ermöglichen, insbesondere von 4-Amino-4-desoxyzuckerderivaten, die als Zwischenprodukte zur Synthese von pharmakologisch wirksamen Verbindungen, wie Antibiotika, nützlich sind.

Es hat sich gezeigt, daß man in üblicher Weise erhaltene Triflatderivate von Hydroxyverbindungen mit Ammoniak — einem ungeladenen Nukleophil — unter wasserfreien Bedingungen und in einem organischen Lösungsmittel — direkt zu den angestrebten premären Aminoverbindungen umsetzen kann, und daß in diesem Falle die Triflate nicht zuvor stabilisiert werden müssen. Im Falle von Zuckertriflatderivaten erhält man direkt die gewünschten Aminozucker.

Gegenstand der Erfindung ist somit ein Verfahren zur Herstellung von primären Aminoverbindungen der allgemeinen Formel II

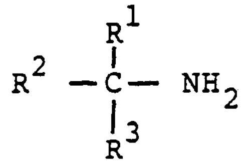

(II)

worin $R^1$, $R^2$ und $R^3$ gleich oder unterschiedlich sein können und beliebige organische Reste mit Ausnahme einer ungeschützten primären oder sekundären Aminogruppe sein können, oder worin zwei der Substituenten mit dem Kohlenstoffatom oder dem Stickstoffatom, an das sie gebunden sind, miteinander unter Ringbildung, wobei der Ring auch ein oder mehrere Heteroatome enthalten kann, verbunden sein können, oder worin die Substituenten zusammen mit dem Kohlenstoffatom oder dem Stickstoffatom, an das sie gebunden sind, auch Teile eines carbocyclischen oder heterocyclischen Ringsystems sein können, bei dem

eine Hydroxylverbindung der allgemeinen Formel III

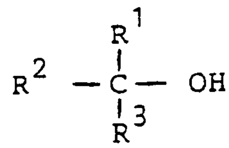

(III)

worin $R^1$, $R^2$ und $R^3$ die oben angegebenen Bedeutungen besitzen, mit Trifluormethansulfonsäureanhydrid der allgemeinen Formel IV

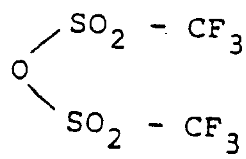

(IV)

bei wasserfreien Bedingungen in das entsprechende Triflat der allgemeinen Formel V

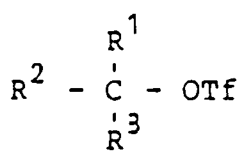

(V)

worin Tf den Rest $—SO_2—CF_3$ bedeutet, umgesetzt wird. Das Verfahren ist dadurch gekennzeichnet, daß man anschließend das Triflatderivat der allgemeinen Formel V unter wasserfreien Bedingungen in einem organischen Lösungsmittel mit Ammoniak umsetzt und die erhaltene Aminoverbindung der allgemeinen Formel I isoliert.

Beispiele für beliebige organische Reste, die durch $R^1$, $R^2$ und $R^3$ wiedergegeben werden, sind Alkyl-, Alkenyl- Alkylaryl-, Cycloalkyl- oder Arylgruppen, die sehr stark variiert werden können und beispielsweise Etherreste, Thioetherreste, Sulfonsäurereste, Nitroreste, Alkylsilanreste, Cycloalkinreste, Cycloalkenreste oder Cycloalkanreste tragen. Diese Reste können jeweils durch Halogen- oder Carboxygruppen substituiert sein. $R^1$, $R^2$ und $R^3$ können weiterhin eine substituierte Alkenylgruppe, eine Alkinyl-, Haloalkyl-, Nitroalkyl- oder Nitroarylgruppe, aromatische Carbonsäurereste oder ihre Derivate, wie Ester- oder Säureamidreste, ein Halogenatom, eine heterocyclische Gruppe, Zuckerreste, Aminosäurereste, Steroidreste, Terpenreste, Ketonreste etc. bedeuten.

Bevorzugte Gruppen für $R^1$, $R^2$ und $R^3$ sind beispielsweise gesättigte oder ungesättigte aliphatische, alicyclische oder cycloaliphatische Kohlenwasserstoffe mit 4 bis 120 (vorzugsweise 6 bis 40) Kohlenstoffatomen. Die Kohlenswasserstoffe können gegebenenfalls durch Sauerstoffatome, Schwefelatome, Sulfinylgruppen, Sulfonylgruppen, Sulfonyloxygruppen, tertiäre Aminogruppen und/oder Amidgruppen unterbrochen sein. Sie können unsubstituiert sein oder beispielsweise durch Oxogruppen, Halogenatome oder Arylgruppen substituiert sein. Derartige Reste sind beispielsweise aliphatische, alicyclische oder cycloaliphatische Ether- oder Esterreste, Reste von gesättigten oder ungesättigten Steroiden, die gegebenenfalls durch Oxogruppen, Halogenatome, $C_1—C_6$-Alkoxygruppen, $C_2—C_8$-Acyloxygruppen substituiert sein können, Zuckerreste, in denen alle Hydroxygruppen mit Ausnahme der umzusetzenden in üblicher Weise geschützt sind, Arylalkylreste, in denen die Arylgruppe (vorzugsweise die Phenylgruppe oder die Naphthylgruppe) in üblicher Weise substituiert sein kann (beispielsweise durch $C_1—C_6$-Alkyl, $C_1—C_6$-Alkoxy, $C_1—C_8$-Acyloxy, Halogen- oder Nitrogruppen) oder Alkaloidreste.

Der Substituent $R^1$ ist bevorzugt eine niedere Alkylgruppe mit 1 bis 4 Kohlenstoffatomen, besonders bevorzugt ein Wasserstoffatomen.

Der Substituent $R^2$ kann als gesättigter oder ungesättigter aliphatischer, alicyclischer oder cycloaliphatischer Kohlenwasserstoffrest gegebenenfalls durch Sauerstoffatome oder Amidgruppen unterbrochen und beispielsweise durch Oxogruppen, Halogenatome oder Arylgruppen substituiert sein. Als cyclische Kohlenwasserstoffe kommen vorzugsweise solche mit einer Ringgröße von 3 bis 16 Kohlenstoffatomen, insbesondere solche mit einer Ringgröße von 5 oder 6 Kohlenstoffatomen, in Betracht. Durch Oxygruppen unterbrochene und/oder durch Oxogruppen substituierte Kohlenwasserstoffe sind beispielsweise Kohlenwasserstoffe, die Ethergruppen, Estergruppen, Carbonylgruppen, Oxirangruppen oder Ketalgruppen, wie zum Beispiel Isopropylidenketale, tragen. Sind die Kohlenwasserstoffe durch Halogenatome substituiert, so kann der Fall eintreten, daß auch die Halogenatome zumindest partiell gegen Aminogruppen ausgetauscht werden. Als durch Arylgruppen und/oder Oxogruppen substituierte und gegebenenfalls durch Sauerstoffatome unterbrochene Kohlenwasserstoffreste seien beispielsweise genannt: Benzylgruppen, Benzyloxygruppen, Benzoylgruppen, oder Benzoyloxygruppen, welche wie oben erwähnt substituiert sein können. In der Bedeutung der Arylgruppe ist der Substituent $R^2$ vorzugsweise eine Phenyl- oder Naphthylgruppe, die wie oben beschrieben substituiert sein kann.

Der Substituent $R^3$ besitzt die gleiche Bedeutung wie $R^1$ oder $R^2$. Der Substituent $R^3$ kann auch gemeinsam mit $R^2$ und dem $R^2$ und $R^3$ verbindenden Kohlenstoffatom einen alycyclischen Kohlenwasserstoffrest, der gegebenenfalls durch Sauerstoffatome unterbrochen sein kann, bilden. Beispiele hierfür sind der Tetrahydrofuranylrest oder der Tetrahydropyranylrest, gesättigt oder ungesättigt. Die gebildeten Reste können monocyclisch oder, wie beispielsweise bei Steroidresten, polycyclisch sein. Dieser alicyclische Kohlenwasserstoffrest kann in gleicher Weise substituiert sein wie oben für die aliphatischen Substituenten angegeben.

Nach dem erfindungsgemäßen Verfahren lassen sich beispielsweise 4-Amino-4-desoxyzucker der allgemeinen Formel VI

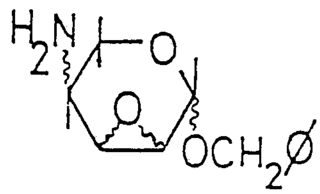

(VI)

herstellen, indem man einen Benzyl-2,3-anhydrozucker der allgemeinen Formel VII

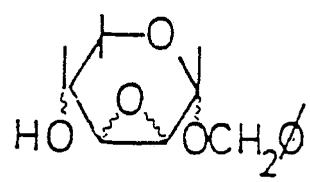

(VII)

mit Trifluormethansulfonsäureanhydrid der Formel III

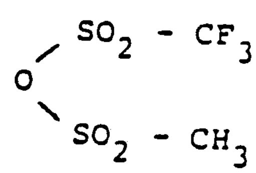

(III)

bei wasserfreien Bedingungen in das entsprechende Triflat der allgemeinen Formel VIII

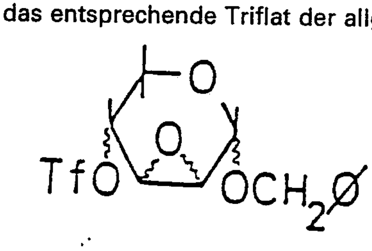

(VIII)

überführt und das Triflatderivat unter wasserfreien Bedingungen in einem organischen Lösungsmittel mit Ammoniak umsetzt und den erhaltenen 4-Amino-4-desoxyzucker der Formel VI isoliert.

Bei der ersten Stufe des erfindungsgemäßen Verfahrens wird eine Hydroxylverbindung in einem organischen Lösungsmittel gelöst oder suspendiert. Das Lösungsmittel sollte möglichst wasserfrei sein. Man arbeitet bei dieser Stufe unter Wasserausschluß. Als Lösungsmittel können beliebige Lösungsmittel verwendet werden, solange sie mit den Ausgangsmaterialien und den Reaktionsprodukten nicht reagieren. Vorzugsweise verwendet man als Lösungsmittel inerte organische Lösungsmittel, beispielsweise Halogenkohlenwasserstoffe, wie Dichlormethan oder Chloroform, aromatische Kohlenwasserstoffe, wie Benzol,

Xylol oder Toluol, gesättigte Kohlenwasserstoffe, DMSO, Ether, wie Diethylether, Diisopropylether, 1,2-Dimethoxyethan, Tetrahydrofuran oder Dioxan, oder dipolare aprotische Lösungsmittel, wie Dimethylformamid oder Hexamethylphosphorsäuretriamid.

Die Lösung oder Suspension der Hydroxylverbindung wird auf eine Temperatur im Bereich von −20°C bis +10°C, vorzugsweise auf eine Temperatur im Bereich von 0 bis 5°C, gekühlt.

Zu der gekühlten Lösung oder Suspension gibt man portionsweise unter Rühren das Trifluormethansulfonsäureanhydrid, ebenfalls gelöst in einem absoluten Lösungsmittel, hinzu. Das Lösungsmittel zum Lösen des Trifluormethansulfonsäureanhydrids kann das gleiche sein wie das, welches man zum Lösen der Hydroxylverbindung verwendet hat, es kann jedoch auch ein anderes sein. Bei der Zugabe, vorzugsweise der tropfenweisen Zugabe, wird die Reaktionstemperatur innerhalb eines Bereichs von −20°C bis +10°C, vorzugsweise bei 0 bis 5°C, gehalten. Die Reaktion läuft praktisch momentan ab. Zur Beendigung der Reaktion rührt man im allgemeinen während einer Zeit von 30 min bis 10 h. Es ist jedoch üblicherweise nicht erforderlich, die Reaktionszeit zu lange auszudehnen, und ein Nachrühren während einer Zeit von 30 min bis 2 h ist bevorzugt. Die Aufarbeitung des Reaktionsprodukts kann in unterschiedlicher Weise erfolgen. Beispielsweise kann man das Reaktionsprodukt auf zerkleinertes Eis geben, welches eine geringe Menge an einem Alkalibicarbonat, wie Natrium- oder Kaliumbicarbonat, enthält. Man schüttelt das erhaltene Gemisch kurz kräftig durch, trennt die organische Phase von der wäßrigen Phase ab und wäscht mit eiskalter 0,5 bis 2N wäßriger Säure, beispielsweise Salzsäure, nach. Die organische Phase wird dann in an sich bekannter Weise, beispielsweise über Natriumsulfat, getrocknet und eingeengt.

Man erhält das gewünschte Triflat als Öl oder als kristalline Substanz. Eine Reinigung des erhaltenen Rohprodukts durch Säulenchromatographie und/oder Kristallisation ist möglich, bringt aber in der Regel keine Vorteile.

Das erhaltene Triflat wird dann wieder in einem absoluten Lösungsmittel gelöst, wobei als Lösungsmittel die oben erwähnten Lösungsmittel verwendet werden können. Zu der Lösung gibt man eine 0,5 bis 2N Lösung von Ammoniak oder einer primären oder sekundären Aminoverbindung in einem absoluten Lösungsmittel, wobei als Lösungsmittel ebenfalls die oben erwähnten Lösungsmittel verwendet werden können. Das erhaltene Reaktionsgemisch wird dann während einer Zeit von 2 bis 24 h bei einer Temperatur von 20°C bis zur Rückflußtemperatur des Reaktionsgemisches gerührt. Anschließend wird das Reaktionsgemisch mit einer wäßrigen Alkalilösung und Wasser gewaschen, und die organische Phase wird dann getrocknet und in an sich bekannter Weise, zum Beispiel durch Destillation, Abdestillieren des Lösungsmittels, Chromatogaphie etc., gereinigt. Man erhält die gewünschte Aminoverbindung im allgemeinen in hoher Ausbeute.

Da der Austausch der Trifluormethylsulfonyloxygruppe gegen die Aminogruppe von Ausnahmen abgesehen unter Inversion abläuft, eignet sich das erfindungsgemäße Verfahren unter anderem recht gut zur Herstellung optisch aktiver Aminoverbindungen aus optisch aktiven Hydroxyverbindungen. Dies ist insofern von Bedeutung, als man optisch aktive Hydroxyverbindungen meist sehr gut durch enzymatische oder mikrobiologische Reduktion der entsprechenden Ketoverbindungen herstellen kann.

Anhand der folgenden Formelschemata wird das erfindungsgemäße Verfahren näher erläutert.

1. Herstellung von Benzyl-2,3-anhydro-4-amino-4-deoxy-α-D-lyxopyranosid

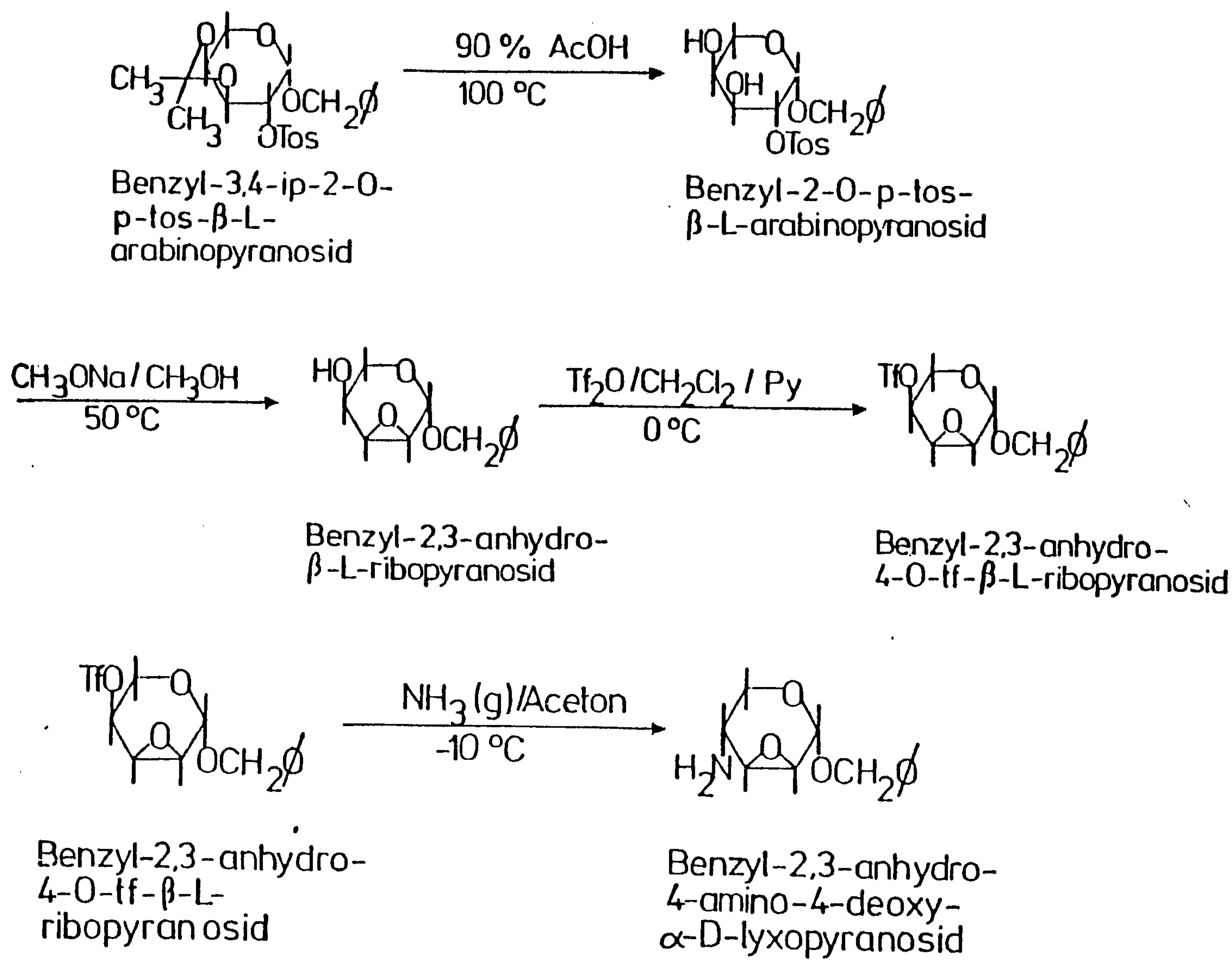


Benzyl-3,4-ip-2-O-p-tos-β-L-arabinopyranosid

Benzyl-2-O-p-tos-β-L-arabinopyranosid

Benzyl-2,3-anhydro-β-L-ribopyranosid

Benzyl-2,3-anhydro-4-O-tf-β-L-ribopyranosid

Benzyl-2,3-anhydro-4-O-tf-β-L-ribopyranosid

Benzyl-2,3-anhydro-4-amino-4-deoxy-α-D-lyxopyranosid

2. Herstellung von Benzyl-2,3-anhydro-4-deoxy-4-amino-β-L-lyxopyranosid

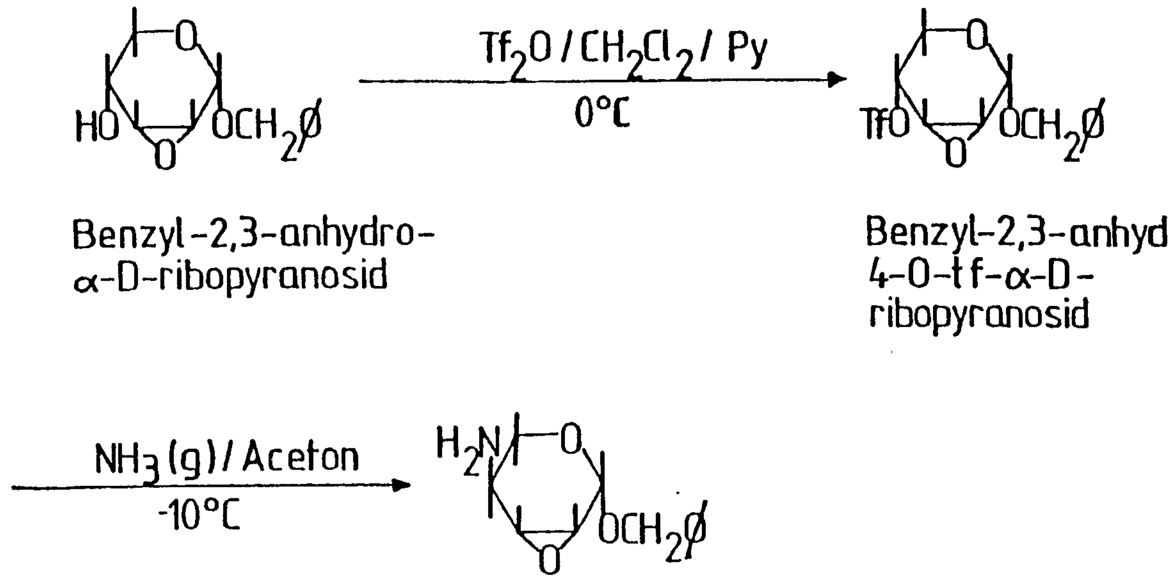


Benzyl-2,3-anhydro-α-D-ribopyranosid

Benzyl-2,3-anhydro-4-O-tf-α-D-ribopyranosid

Benzyl-2,3-anhydro-4-deoxy-4-amino-β-L-lyxopyranosid

3. Herstellung von Benzyl-2,3-anhydro-4-amino-4-deoxy-3-L-ribopyranosid

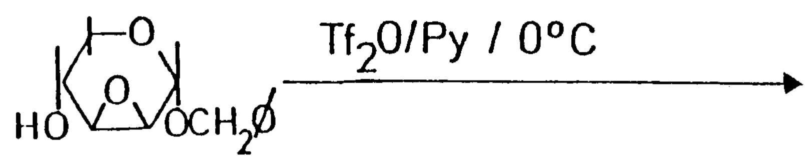


Benzyl-2,3-anhydro-α-D-lyxopyranosid

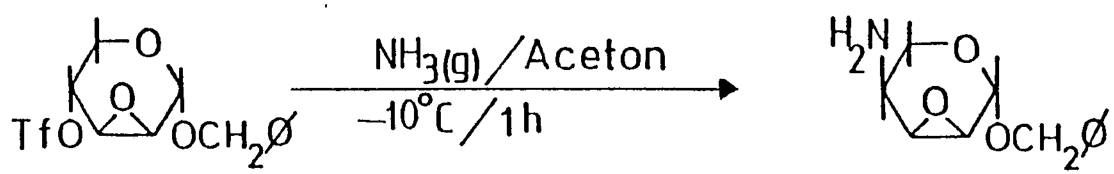


Benzyl-2,3-anhydro-4-O-tf-α-D-lyxopyranosid

Benzyl-2,3-anhydro-4-amino-4-deoxy-3-L-ribopyranosid

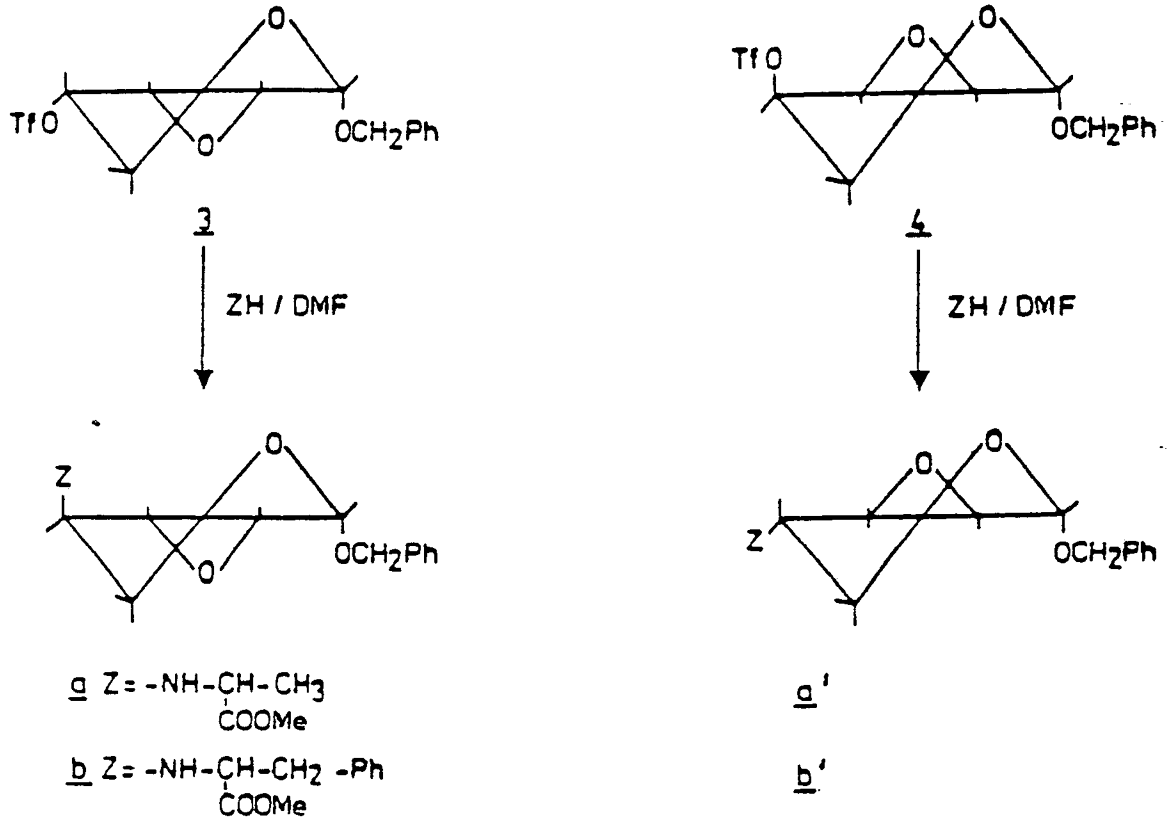


Herstellung von Benzyl-2,3-dideoxy-2-bromo-4-(Ala-$OCH_3$)-β-L-xylo-pyranosid

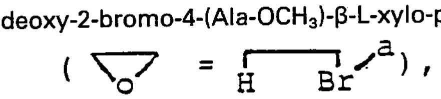

Benzyl-2,3-anhydro-4-deoxy-(Phe-$OCH_3$)-β-L-lyxopyranosid (b), Benzyl-2,3-anhydro-4-deoxy-4-(Ala-O-$CH_3$)-α-D-lyxopyranosid (a') und Benzyl-2,3-anhydro-4-deoxy-(Phe-$OCH_3$)-α-D-lyxopyranosid.

Das erfindungsgemäße Verfahren eignet sich besonders zur Herstellung von Aminosäuren aus den entsprechenden Hydroxycarbonsäuren. Zur Herstellung von Aminosäuren wird die Hydroxygruppe vorzugsweise in die Estergruppe überführt, und dann wird die erfindungsgemäße Reaktion durchgeführt.

Das Verfahren eignet sich ferner, um Aminosäuren mit Triflatverbindungen, z.B. Triflatzuckern, zu einer neuen Verbindungsklasse der Struktur

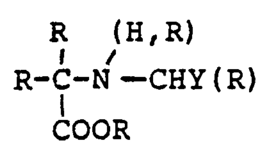

umzusetzen,

wobei R für ein Wasserstoffatom steht oder für eine beliebige organische Gruppe wie Alkyl-, Alkenyl-, Alkylaryl-, Cycloalkyl- oder Arylgruppe, die Etherreste, Thioetherreste, Sulfonsäurereste, Nitroreste, Alkylsilanreste, Cycloalkinreste, Cycloalkenreste oder Cycloalkanreste tragen kann und die jeweils durch Halogen- oder Carboxygruppen substituiert sein kann, oder für eine substituierte Alkenylgruppe, eine Alkinyl-, Haloalkyl-, Nitroalkyl- oder Nitroarylgruppe, aromatische Carbonsäurereste oder ihre Derivate, wie Ester- oder Säureamidreste, ein Halogenatom oder eine Heterocyclische Gruppe, und Y ein Zuckerrest ist.

Die folgenden Beispiele erläutern die Erfindung.

## Beispiel 1

a) 22,3 g 2-Heptanol werden in 500 ml absolutem Dichlormethan gelöst, mit 25 ml absolutem Pyridin versetzt und auf −5°C gekühlt. Dann tropft man unter Rühren eine Lösung von 62,1 g Trifluormethansulfonsäureanhydrid in 500 ml absolutem Dichlormethan zu, wobei die Reaktionstemperatur bei −5°C bis 0°C gehalten wird. Man rührt noch eine weitere Stunde lang und gießt das Reaktionsprodukt in zerkleinertes Eis, welches mit 10 g Natriumbicarbonat versetzt ist.

Man schüttelt das Gemisch kurz kräftig durch, trennt die Dichlormethanphase ab, wäscht sie mit eiskalter 1N wäßriger Salzsäure, verdünnter Natriumbicarbonatlösung und Wasser, trocknet sie über Natriumsulfat und engt die erhaltene Lösung ein. Man erhält so 44,0 g 2-(Trifluormethylsulfonyloxy)-heptan als hellgelbes Öl.

b) 44,0 g des so erhaltenen 2-(Trifluormethylsulfonyloxy)-heptans werden in 500 ml absolutem Chloroform gelöst, mit 500 ml einer 2N Lösung von Ammoniak in absolutem Chloroform versetzt und 16 Stunden lang bei 50°C gerührt. Dann wäscht man das Reaktionsgemisch mit wäßriger Natriumhydrogencarbonatlösung und Wasser, trocknet sie über wasserfreiem Magnesiumsulfat und unterwirft sie einer fraktionierten Destillation. Die bei 132 bis 150°C destillierende Fraktion wird nochmals fraktioniert, und man erhält 15,4 g 2-Heptylamin (= Tuamin) vom Siedepunkt 140 bis 144°C.

Beispiel 2

a) 2,7 g 1-Phenyl-2-propanol werden in 50 ml absolutem Chloroform gelöst, mit 5 ml Pyridin versetzt, auf −5°C gekühlt und tropfenweise unter Rühren mit einer Lösung von 5,7 g Trifluormethansulfonsäureanhydrid in 50 ml absolutem Chloroform versetzt, so daß die Temperatur des Reaktionsgemisches 0°C nicht übersteigt. Man rührt noch eine Stunde lang bei 0°C, wäscht die Reaktionsmischung mit eiskalter 1N Schwefelsäure, Bicarbonatlösung und Wasser und trocknet sie über Magnesiumsulfat.

b) Die so erhaltene Lösung des 1-Phenyl-2-trifluormethylsulfonyloxypropans wird mit 200 ml einer 2N Ammoniaklösung in absolutem Chloroform versetzt und 16 Stunden lang bei 50°C gerührt. Dann wird die Reaktionsmischung mit Natriumbicarbonatlösung und Wasser gewaschen und das Lösungmittel im Vakuum abdestilliert. Der Rückstand wird aus Schwefelsäure enthaltendem Ethanol umkristallisiert, und man erhält 2,6 g 1-Phenyl-2-propylaminsulfat (= Amphetaminsulfat) vom Zersetzungspunkt oberhalb 300°C.

Beispiel 3

Unter den Bedingungen des Beispiels 2 werden 3,5 g 2-Hydroxy-3-phenyl-propionsäuremethylester umgesetzt und aufbereitet. Das erhaltene Rohprodukt wird fraktioniert, und man erhält 2,0 g 2-Amino-3-phenylpropionsäuremethylester (= Phenylalaninmethylester) vom Siedepunkt 140 bis 144°C bei 16 mbar.

Beispiel 4

Unter den Bedingungen des Beispiels 2 werden 3,1 g 12-Hydroxy-9-octadecensäure-methylester umgesetzt, aufbereitet, und man erhält 2,9 g 12-Amino-9-octadecensäuremethylester als Rohprodukt.

Das Produkt hat die typischen Eigenschaften eines Kationentensids.

Beispiel 5

Unter den Bedingungen des Beispiels 2 werden 4,0 g Cycloheptylalkohol umgesetzt und aufbereitet. Das erhaltene Rohprodukt wird im Vakuum fraktioniert, und man erhält 2,9 g Cycloheptylamin vom Siedepunkt 49 bis 53°C bei 14,6 mbar.

Beispiel 6

Unter den Bedingungen des Beispiels 2 werden 3,8 g 4-Chlorbenzylalkohol umgesetzt und aufbereitet. Das erhaltene Rohprodukt wird im Vakuum fraktioniert, und man erhält 2,3 g 4-Chlorbenzylamin vom Siedepunkt 102 bis 107°C bei 18,6 mbar.

Beispiel 7

Unter den Bedingungen des Beispiels 2 werden 2,5 g 3β-Acetoxy-7β-hydroxy-5-cholesten umgesetzt und aufbereitet. Das erhaltene Rohprodukt wird eine Stunde lang mit einer Mischung aus 3 ml konzentrierter Salzsäure und 30 ml Ethanol erhitzt, im Vakuum eingeengt, und der Rückstand in Ethanol, dem etwas Salzsäure zugesetzt ist, umkristallisiert. Man erhält so 1,7 g 7α-Amino-3β-hydroxy-cholesten-hydrochlorid vom Zersetzungspunkt 270°C.

Beispiel 8

Unter den Bedingungen des Beispiels 2 werden 2,1 g 3α-20β-Diacetoxy-pregnan-11β-ol umgesetzt und aufbereitet. Das erhaltene Rohprodukt wird eine Stunde lang mit 25 ml Ethanol, welches 2 ml konzentrierte Schwefelsäure enthält, unter Rückfluß erhitzt, mit Ethanol auf 100 ml verdünnt, die Schwefelsäure durch Schütteln mit Amberlite® IR-A 400 entfernt und die Lösung im Vakuum eingeengt. Man kristallisiert den Rückstand aus Dioxan um und erhält so 1,3 g 11α-Amino-pregnan-3α,20β-diol vom Schmelzpunkt 188 bis 190°C.

Beispiel 9

2,8 g 2-(1-Acetyl-3-indolyl)-ethanol werden unter den Bedingungen des Beispiels 2 umgesetzt und aufbereitet. Das erhaltene Rohprodukt wird mit 50 ml 6N Salzsäure eine Stunde lang erhitzt, die Mischung im Vakuum eingeengt und der Rückstand aus Ethanol/Ethylacetat unter Zusatz von etwas Salzsäure umkristallisiert. Man erhält so 1,6 g 2-(3-Indolyl)-ethylamin-hydrochlorid (Tryptamino-hydrochlorid) vom Schmelzpunkt 246 bis 247,5°C.

Beispiel 10

Unter den Bedingungen des Beispiels 2 werden 2,8 g ω-Hydroxyacetophenon umgesetzt, aufbereitet, und man erhält nach Umkristallisation aus salzsäurehaltigem Ethanol 22 g ω-Aminoacetophenon-hydrochlorid (Phenylacylamin-hydrochlorid) vom Zersetzungspunkt 184 bis 186°C.

Beispiel 11

a) Benzyl-2,3-anhydro-4-triflyl-α-D-lyxopyranosid

2,2 g (10 mmol) Benzyl-2,3-anhydro-α-D-lyxopyranosid werden in 50 ml absolutem Methylendichlorid gelöst. Dazu gibt man 1 ml absolutes Pyridin und kühlt auf 0°C. Eine lösung von 1,6 ml (10 mmol) Trifluormethansulfonsäureanhydrid in 50 ml absolutem Methylendichlorid wird tropfenweise unter konstantem Rühren zugegeben, wobei die Temperatur bei 0°C gehalten wird. Das Reaktionsgemisch wird weitere 30 min bei dieser Temperatur gerührt, in zerkleinertes Eis, welches 200 mg Natriumbicarbonat enthält, gegeben, und dann werden 250 ml Methylendichlorid hinzugefügt, und die Schichten getrennt.

Die organische Phase wird schnell mit 1N Chlorwasserstoffsäure, Wasser, verdünnter Natriumbicarbonatlösung und erneut mit Wasser gewaschen. Der nach dem Trocknen und Entfernen des Lösungsmittels bei verringertem Druck erhaltene Rückstand wird in Ethanol aufgenommen und gekühlt. Es kristallisieren 2,97 g (90%) der oben genannten Titelverbindung aus.

Fp.: 32°C, $[\alpha]_D^{20}$ = 72,2° (c = 0,8, Chloroform).

IR (KBr): 1422, 1217, 1145 ($OSO_2$), 1250 $cm^{-1}$ (CF).

MS: 354 ($M^+$), 246 ($M—PhCH_2OH$), 204 ($M—CF_3SO_2OH$), 133 ($CF_3SO_2^+$), 108 ($PhCH_2OH^+$), 91 ($PhCH_2^+$).

$^1H$—NMR (90 MHz, $CDCl_3$): δ 7,26 (5H; Phenylprotonen), 5,02 (1H, m; H—4), 4,87 (1H, S; H—1), 4,59 (2H, dd; J=12,5Hz, Benzylprotonen), 3.65 (1H, m; H—5), 3,33 (1H, d, J=3,44; H—3), 3,14 (1H, d, J=3,76; H—2).

$C_{13}H_{13}F_3O_6S$ (354,29)

| | | | |
|---|---|---|---|
| Berechnet: | C 44,07 | H 3,71 | S 9,05 |
| Gefunden: | C 43,79 | H 3,79 | S 9,10. |

b) Benzyl-2,3-anhydro-4-amino-4-desoxy-β-L-ribopyranosid

1,1 g (3 mmol) der Titelverbindung von a) werden in 20 ml absolutem Chloroform gelöst. Dazu werden 20 ml 2N Lösung von Ammoniak in absolutem Chloroform zugegeben, und dann wird bei 50°C während 5 Stunden gerührt. Das Lösungsmittel wird im Vakuum entfernt, der Rückstand wird in Ethylacetat aufgenommen und erschöpfend mit 1N Essigsäure extrahiert. Die wäßrige Fraktion wird gefriergetrocknet, der Rückstand wird mit einer verdünnten Lösung von Natriumbicarbonate gewaschen und mit Ethylacetat extrahiert. Nach dem Trocknen und dem Entfernen des Lösungsmittels im Vakuum erhält man 420 mg (63,2%) der gewünschten Verbindung.

Fp.: 55°C, $[\alpha]_D^{20}$ = +54,4° (c = 3, Chloroform).

IR (KBr): Doppelbande zwischen 3300—3500 ($NH_2$-Gruppen) und ausgeprägte Bande zwischen 1560—1650 $cm^{-1}$ ($NH_2$-Gruppe).

FDMS ($CHCl_3$): 221 ($M^+$), 113 ($M—PhCH_2OH$), 108 ($PhCH_2OH^+$), 91 ($PhCH_2^+$).

$^1H$—NMR (90 MHz, $CDCl_3$): δ 7,38 (5H, S; Phenylprotonen), 4,99 (1H, S; H—1), 4,71 (2H, dd, $J_{gem}$ = 12,54Hz; Benzylprotonen), 3,92 (1H, q, J=5,14Hz, J=13,1Hz; H—5), 3,49 (1H, t, J=4,12Hz; H—4), 3,25 (1H, m; H—2, H—3), 1,80 (2H, S; $NH_2$-Protonen).

$C_{12}H_{15}NO_3$ (221,28)

| | | | |
|---|---|---|---|
| Berechnet: | C 65,13 | H 6,85 | N 6,33 |
| Gefunden: | C 64,91 | H 6,33 | N, 6,41. |

c) Umsetzung mit gasförmigem Ammoniak in Aceton

1,1 g (3 mmol) Benzyl-2,3-anhydro-4-triflyl-α-D-lyxopyranosid in 25 ml absolutem Aceton werden auf −10°C gekühlt, und dann wird ein langsamer Strom von gasförmigem Ammoniak unter konstantem Rühren während 1 Stunde eingeleitet. Das Lösungsmittel wird im Vakuum entfernt. Der Rückstand wird in Ethylacetat aufgenommen und erschöpfend mit 1N Essigsäure unter Zugabe von Petrolether extrahiert.

Die saure Schicht wird mit konzentriertem Ammoniak bis zur basischen Reaktion versetzt. Dann wird Kochsalz zugegeben, und es wird mit Ether extrahiert. Die Etherschicht wird über wasserfreiem Natriumsulfat getrocknet und vom Lösungsmittel befreit. Das erhaltene farblose Öl wird mehrere Tage in einem Eisschrank aufbewahrt. Danach kristallisiert das Benzyl-2,3-anhydro-4-amino-4-desoxy-β-L-ribopyranosid in Form einer amorphen Masse aus, welche mit Hilfe von Ether/Petrolether (1:1) abfiltriert wird.

Die Mutterlauge wird vom Lösungsmittel befreit und der Rückstand über Silicagel (Lobar® B, Si 60, Merck) unter Verwendung eines Lösungsmittelsystems Chloroform/Methanol (98:2) als Eluierungsmittel chromatographiert. Aus Ethylacetat kristallisiert eine zweite Charge der Titelverbindung (Gesamtausbeute 498 mg; 75%) aus.

Beispiel 12

a) Benzyl-2,3-anhydro-4-triflyl-α-D-ribopyranosid

2,2 g (10 mmol) Benzyl-2,3-anhydro-α-D-ribopyranosid werden mit Trifluormethansulfonsäureanhydrid wie in Beispiel 11 beschrieben umgesetzt. Aus dem feuchten Ethanol kristallisieren 3,02 g (80,3%) der gewünschten Titelverbindung aus.

Fp.: 66—67°C, $[\alpha]_D^{20}$ = +133° (c = 1, Chloroform).

$^1$H—NMR (90 MHz, $CDCl_3$): δ 7,31 (5H, S; Phenylprotonen), 5,19 (1H, q, J=6,75 und J=8,07; H—4), 4,94 (1H, d, J=2,5Hz; H—1), 4,65 (2H, dd, $J_{gem}$=12,5Hz; Benzylprotonen), 4,08—3,55 (3H, m; H—2, H—3, H—5).

$C_{13}H_{13}F_3O_6S$ (354,29)

Berechnet: C 44,07 H 3,71 S 9,05
Gefunden: C 44,11 H 3,64 S 9,03.

b) Benzyl-2,3-anhydro-4-amino-4-desoxy-β-L-lyxopyranosid

1,1 g (3 mmol) der gemäß a) erhaltenen Verbindung werden mit 2N Ammoniaklösung in Chloroform wie in Beispiel 11 beschrieben umgesetzt. Man erhält 250 mg (52,7%) der Titelverbindung.

Fp.: 37—38°C, $[\alpha]_D^{20}$ = +105° (c = 1, Chloroform).

$^1$H—NMR (90 MHz, $CDCl_3$): δ 7,36 (5H, S; Phenylprotonen), 5,02 (1H, d, J=2,91; H—1), 4,69 (2H, dd, $J_{gem}$=12,54Hz; Benzylprotonen), 4,05 (1H, q, J=2,94Hz, $J_{gem}$=10,5Hz; H—5), 3,33 (1H, S; H—2), 3,29 (1H, S; H—3), 3,20 (1H, t, J=2,93Hz; H—4), 1,76 (2H, S; $NH_2$-Protonen).

$C_{12}H_{15}NO_3$ (221,28)

Berechnet: C 65,13 H 6,85 N 6,33
Gefunden: C 65,27 H 6,73 N 6,19.

c) Arbeitet man auf gleiche Weise wie in Beispiel 11 c) beschrieben, so erhält man den entsprechenden Aminozucker in 63%iger Ausbeute.

Beispiel 13

a) Benzyl-2,3-anhydro-4-triflyl-β-L-ribopyranosid

2,2 g (10 mmol) Benzyl-2,3-anhydro-β-L-ribopyranosid werden mit Trifluormethansulfonsäureanhydrid wie in Beispiel 11 beschrieben umgesetzt. Aus dem feuchten Ethanol kristallisieren 3,49 g (96%) der Titelverbindung aus.

Fp.: 83—84°C, $[\alpha]_D^{20}$ = 17° (c = 1, Chloroform).

$^1$H—NMR (90 MHz, $CDCl_3$): δ 7,35 (5H, S; Phenylprotonen), 5,16 (1H, m; H—4), 5,09 (1H, S; H—1), 4,67 (2H, dd, $J_{gem}$=12,5Hz; Benzylprotonen), 4,15—3,63 (1H, m, J = 4,1 und 13,3; H—5), 3,56 (1H, d, J=3,52; H—3), 3,28 (1H, d, J=3,81; H—2).

$C_{13}H_{13}F_3O_6S$ (354,29)

Berechnet: C 44,07 H 3,71 S, 9,05
Gefunden: C 43,89 H 3,58 S 9,13.

b) Benzyl-2,3-anhydro-4-amino-4-desoxy-α-D-lyxopyranosid

1,1 g (3 mmol) der gemäß a) erhaltenen Verbindung werden mit 2N Ammoniaklösung in Chloroform, wie in Beispiel 11 beschrieben, umgesetzt, und man erhält 520 mg (75,7%) der Titelverbindung.

Fp.: 48—49°C, $[\alpha]_D^{20}$ = +91° (c = 1, Chloroform).

$^1$H—NMR (90 MHz, $CDCl_3$): δ 7,36 (5H, S; Phenylprotonen), 4,98 (1H, S; H—1), 4,68 (2H, dd, $J_{gem}$=12,54Hz; Benzylprotonen), 3,56 (1H, q, J=4,69Hz, $J_{gem}$=10,02Hz; H—5), 3,35 (1H, S; H—2), 3,24 (1H, S; H—3), 3,14 (1H, t, J=4,7Hz; H—4), 1,81 (2H, S; $NH_2$-Protonen).

$C_{22}H_{15}NO_3$ (221,28)

Berechnet: C 65,13 H 6,85 N 6,33
Gefunden: C 65,01 H 6,72 N 6,14.

c) Arbeitet man auf gleiche Weise wie in Beispiel 11 c) beschrieben, so erhält man den entsprechenden Aminozucker in 79,6%iger Ausbeute.

Beispiel 14

Synthese der Verbindungen a, b, a', b'

Allgemeines Verfahren

Die in der folgenden Tabelle aufgeführten Verbindungen a, b, a' und b' wurden nach dem folgenden allgemeinen Verfahren synthetisiert:

Ein mmol des Zuckertriflats wurde in 4 ml reinem Dimethylformamid gelöst. 2,5 mmol des entsprechenden Aminoesters wurden bei −20°C zugegeben. Das Reaktionsgemisch wurde langsam auf Zimmertemperatur erwärmt. Es wurden 5 Stunden kontinuierlich gerührt. Die Reaktion wurde mit Dünnschichtchromatographie verfolgt, wobei als Lösungsmittelsystem Aceton/Dichlormethan/Toluol (1:1:1) verwendet wurde.

Das Lösungsmittel wurde im Hochvakuum entfernt, wobei das als Ausgangsmaterial verwendete Triflat ebenfalls mit entfernt wurde. Der Rückstand wurde in Ethylacetat aufgenommen, wiederholt mit Natriumcarbonatlösung und Wasser gewaschen, über Natriumsulfat getrocknet und vom Lösungsmittel befreit.

Die Verbindung wurden durch Säulenchromatographie unter Verwendung des gleichen oben erwähnten Lösungsmittelsystems in reiner Form isoliert.

Gemäß einem modifizierten Verfahren wurde DMF durch Acetonitril mit hohem Reinheitsgrad ersetzt. Dadurch konnte das Lösungsmittel noch leichter entfernt werden und die Bildung von Nebenprodukten wurde vollständig vermieden. Die Reaktionen wurden bei Zimmertemperatur während 48 Stunden ausgeführt, und man erhielt erhöhte Ausbeuten an den Kupplungsprodukten.

TABELLE

Physikalische Eigenschaften und analytische Werte der hergestellten Produkte

| Verb. Nr. | Name der Verbindung | %-Ausbeute | Fp °C | [α]25°(°)D | Summenformel |
|---|---|---|---|---|---|
| a | Benzyl-2,3-dideoxy-2-bromo-4-(Ala-$OCH_3$)-β-L-lyxopyranosidhydrogenbromid | 46 | 180 (D-Et) | +41,7 | $C_{16}H_{23}NO_5Br_2$ |
| b | Benzyl-2,3-anhydro-4-deoxy-4-(-Phe-$OCH_3$)-β-L-lyxopyranosidhydrogentoluol-p-sulfonat | 68 | 163—164 (M-Et) | +48,6[d] | $C_{29}H_{33}NO_8S$ |
| a' | Benzyl-2,3-anhydro-4-deoxy-4-(-Ala-$OCH_3$)-α-D-lyxopyranosid | 58 | 142 (E-Et) | +51,5 | $C_{23}H_{29}NO_8S$ |
| b' | Benzyl-2,3-anhydro-4-deoxy-4-(-Phe-$OCH_3$)-α-D-lyxopyranosidhydrogentoluol-p-sulfonat | 54 | 134 | +52,5 | $C_{29}H_{33}NO_8S$ |

*Analysenergebnisse*

| | | | | |
|---|---|---|---|---|
| Verbindung a | | | | |
| $C_{16}H_{23}NO_5Br_2$ | berechnet: | C 40,95 | H 4,94 | N 2,98 |
| | gefunden: | C 40,54 | H 4,95 | N 2,99 |
| Verbindung b | | | | |
| $C_{29}H_{33}NO_8S$ | berechnet: | C 62,86 | H 5,98 | N 2,52 |
| | gefunden: | C 62,98 | H 6,05 | N 2,53 |
| Verbindung a' | | | | |
| $C_{23}H_{29}NO_8S$ | berechnet: | C 57,60 | H 6,10 | N 2,92 |
| | gefunden: | C 58,03 | H 6,32 | N 2,96 |
| Verbindung b' | | | | |
| $C_{29}H_{33}NO_8S$ | berechnet: | C 62,68 | H 5,98 | N 2,52 |
| | gefunden: | C 63,24 | H 5,91 | N 2,50 |

**Patentansprüche**

1. Verfahren zur Herstellung von Aminoverbindungen der allgemeinen Formel II

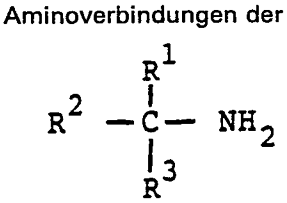

(II)

worin $R^1$, $R^2$ und $R^3$ gleich oder unterschiedlich sein können und beliebige organische Reste mit Ausnahme einer primären oder sekundären ungeschützten Aminogruppe sein können, oder worin zwei der Substituenten mit dem Kohlenstoffatom oder dem Stickstoffatom, an das sie gebunden sind, miteinander unter Ringbildung, wobei der Ring auch ein oder mehrere Heteroatome enthalten kann, verbunden sein

können, oder worin die Substituenten zusammen mit dem Kohlenstoffatom oder dem Stickstoffatom, an das sie gebunden sind, auch Teile eines carbocyclischen oder heterocyclischen Ringsystems sein können, bei dem eine Hydroxylverbindung der allgemeinen Formel III

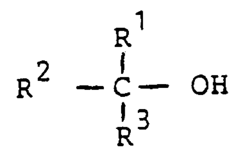

(III)

worin $R^1$, $R^2$ und $R^3$ die oben angegebenen Bedeutungen besitzen, mit Trifluormethansulfonsäureanhydrid unter wasserfreien Bedingungen unter Bildung des Triflatderivats der allgemeinen Formel V

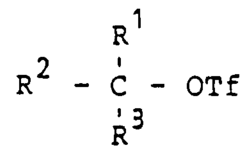

(V)

worin Tf den Rest $—SO_2—CF_3$ bedeutet, umgesetzt wird dadurch gekennzeichnet, daß man anschließend das Triflatderivat der allgemeinen Formel V unter wasserfreien Bedingungen in einem organischen Lösungsmittel mit Ammoniak umsetzt und das Reaktionsprodukt isoliert.

2. Verfahren nach Anspruch 1, dadurch gekennzeichnet, daß man die Umsetzung des Triflatderivats mit Ammoniak bei einer Temperatur im Bereich von 20°C bis zur Rückflußtemperatur des Reaktionsgemisches durchführt.

3. Verfahren nach Anspruch 1 oder 2, dadurch gekennzeichnet, daß man die Umsetzung der Hydroxylverbindung mit Trifluormethansulfonsäureanhydrid bei einer Temperatur im Bereich von −20°C bis +10°C während einer Zeit von 10 Minuten bis zu 8 Stunden durchführt.

4. Verfahren zur Herstellung von Verbindungen der allgemeinen Formel

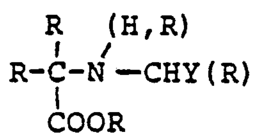

in der R für ein Wasserstoffatom steht oder für eine beliebige organische Gruppe wie Alkyl-, Alkenyl-, Alkylaryl-, Cycloalkyl- oder Arylgruppe, die Etherreste, Thioetherreste, Sulfonsäurereste, Nitroreste, Alkylsilanreste, Cycloalkinreste, Cycloalkenreste oder Cycloalkanreste tragen kann und die jeweils durch Halogen- oder Carboxygruppen substituiert sein kann oder für eine substituierte Alkenylgruppe, eine Alkinyl-, Haloalkyl-, Nitroalkyl- oder Nitroarylgruppe, aromatische Carbonsäurereste oder ihre Derivate, wie Ester- oder Säureamidreste, ein Halogenatom oder eine heterocyclische Gruppe, und Y einen Zuckerrest bedeutet, dadurch gekennzeichnet, daß man einen Triflatzucker der allgemeinen Formel

Y—OTf

in der Tf die $—SO_2—CF_3$ bedeutet, mit einem einer Aminosäure entsprechenden primären oder sekundären Amin der allgemeinen Formel

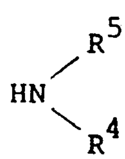

in der $R^4$ und $R^5$ voneinander verschieden sind und die gleiche Bedeutung wie R haben, unter wasserfreien Bedingungen in einem organischen Lösungsmittel umsetzt und das Reaktionsprodukt isoliert.

5. Verfahren nach Anspruch 4, dadurch gekennzeichnet, daß man die Reaktion bei einer Temperatur wie im Bereich von 20°C bis zu Rückflußtemperatur des Reaktionsgemisches durchführt.

6. Verfahren nach Anspruch 5 oder 6, dadurch gekennzeichnet, daß man Benzyl-2,3-dideoxy-2-bromo-4-(Ala-$OCH_3$)-β-L-lyxopyranosidhydrogenbromid herstellt.

7. Verfahren nach Anspruch 5 oder 6, dadurch gekennzeichnet, daß man Benzyl-2,3-anhydro-4-deoxy-4-(-Phe-$OCH_3$)-β-L-lyxopyranosidhydrogentoluol-p-sulfonat herstellt.

8. Verfahren nach Anspruch 5 oder 6, dadurch gekennzeichnet, daß man Benzyl-2,3-anhydro-4-deoxy-4-(-Ala-$OCH_3$)-α-D-lyxopyranosid herstellt.

9. Verfahren nach Anspruch 5 oder 6, dadurch gekennzeichnet, daß man Benzyl-2,3-anhydro-4-deoxy-4-(-Phe-$OCH_3$)-α-D-lyxopyranosidhydrogentoluol-p-sulfonat herstellt.

**Revendications**

1. Procédé de préparation de composés aminés de formule générale II:

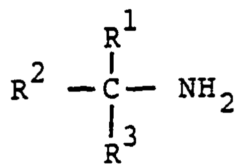

(II)

dans laquelle $R^1$, $R^2$ et $R^3$, identiques ou différents, représentent des groupes organiques quelconques à l'exception d'un groupe aminé primaire ou secondaire non protégé, ou bien dans laquelle deux des substituants sont liés à l'atome de carbone ou l'atome d'azote, sur lequel ils sont liés, pour constituer un cycle renfermant le cas échéant un ou plusieurs hétéroatomes, ou bien dans laquelle les substituants constituent, avec l'atome de carbone ou d'azote sur lequel ils sont liés, également des parties d'un système carbocyclique ou hétérocyclique, selon lequel on fait réagir un composé hydroxylé de formule générale III:

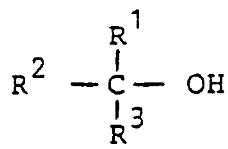

(III)

dans laquelle $R^1$, $R^2$, $R^3$ ont la signification indiquée ci-dessus, avec un anhydride de l'acide trifluorométhane sulfonique dans des conditions anhydres pour former un dérivé trifluoré de formule générale V:

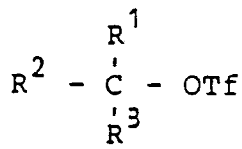

(V)

dans laquelle Tf signifie le groupe $-SO_2-CF_3$, caractérisé en ce qu'on fait réagir ensuite l'ammoniac avec le dérivé trifluoré de formule générale V dans des conditions anhydres dans un solvant organique et en ce qu'on isole le produit de la réaction.

2. Procédé selon la revendication 1, caractérisé en ce qu'on conduit la réaction du dérivé trifluoré avec l'ammoniac à une température comprise entre 20°C et la température de reflux du mélange réactionnel.

3. Procédé selon la revendication 1 ou 2, caractérisé en ce que la réaction du composé hydroxylé avec l'anhydride de l'acide trifluorométhane sulfonique est conduite à une température comprise entre −20 et +10°C pendant 10 minutes jusqu'à 8 heures.

4. Procédé de préparation des composés de formule générale:

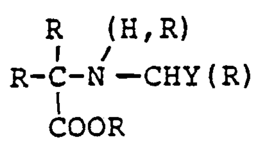

dans laquelle R représente un atome d'hydrogène ou bien un groupe organique quelconque, tel alkyle, alcényle, alkylaryle, cycloalkyle ou aryle, comportant des groupements éther, thioéther, acide sulfonique, nitro, alkylsilane, cycloalkyle, cycloalcényle ou cycloalcane, substitués le cas échéant par des halogènes ou des groupes carboxy, ou bien un groupe alcényle substitué, un groupe alcynile, haloalkyle, nitroalkyle ou nitroaryle, des groupes acides carboxyliques aromatiques ou leurs dérivés tels un reste amide acide ou ester, un atome d'halogène ou un groupe hétérocyclique et y signifie un reste saccharique, caractérisé en ce qu'on fait réagir un sucre trifluoré de formule générale:

$$Y-OTf$$

dans laquelle Tf signifie $-SO_2-CF_3$, avec une amine primaire ou secondaire correspondant à un amino-acide, de formule générale:

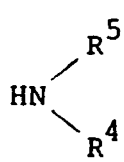

dans laquelle $R^4$ et $R^5$ sont différents l'un de l'autre, et ont la signification de R, dans des conditions exemptes d'eau dans un solvant organique et en ce qu'on isole le produit de la réaction.

5. Procédé selon la revendication 4, caractérisé en ce qu'on conduit la réaction à une température comprise entre 20°C et la température de reflux du mélange réactionnel.

6. Procédé selon la revendication 5, caractérisé en ce qu'on prépare l'hydrogénobromure de benzyl-2,3-didéoxy-2-bromo-4-(Ala-$OCH_3$)-β-L-lyxopyranoside.

7. Procédé selon la revendication 5 ou 6, caractérisé en ce qu'on prépare l'hydrogénotoluol-p-sulfonate de benzyl-2,3-anhydro-4-déoxy-4-(-Phé-$OCH_3$)-β-L-lyxopyranoside.

8. Procédé selon la revendication 5 ou 6, caractérisé en ce qu'on prépare le benzyl-2,3-anhydro-4-déoxy-4-(Ala-$OCH_3$)-α-D-lyxopyranoside.

9. Procédé selon la revendication 5 ou 6, caractérisé en ce qu'on prépare l'hydrogénotoluol-p-sulfonate de benzyl-2,3-anhydro-4-déoxy-4-(-Phé-$OCH_3$)-α-D-lyxopyranoside.

**Claims**

1. A process for the preparation of amino compounds corresponding to the general formula II

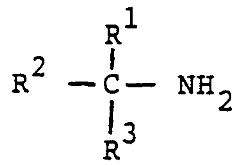

(II)

wherein $R^1$, $R^2$ and $R^3$ may be identical or different and may be any organic groups with the exclusion of a primary or secondary, unprotected amino group or wherein two of the substituents together with the carbon atom or nitrogen atom to which they are attached may be joined together to form a ring which may contain one or more hetero atoms, or wherein the substituents together with the carbon atom or the nitrogen atom to which they are attached may form parts of a carbocyclic or heterocyclic ring system, in which a hydroxyl compound corresponding to the general formula III

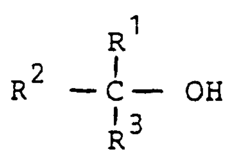

(III)

wherein $R^1$, $R^2$ and $R^3$ have the meanings indicated above is reacted with trifluoromethane sulphonic acid anhydride under anhydrous conditions to form the triflate derivative corresponding to the general formula V

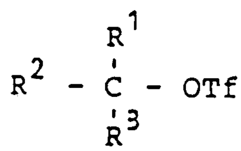

(V)

wherein Tf denotes the group $—SO_2—CF_3$, characterised in that the triflate derivative corresponding to the general formula V is thereafter reacted with ammonia in an organic solvent under anhydrous conditions and the reaction product is isolated.

2. Process according to Claim 1, characterised in that the reaction of the triflate derivative with ammonia is carried out at a temperature in the range of from 20°C to the reflux temperature of the reaction mixture.

3. Process according to Claim 1 or Claim 2, characterised in that the reaction of the hydroxyl compound with trifluoromethane sulphonic acid anhydride is carried out at a temperature from −20°C to +10°C for a period from 10 minutes to 8 hours.

4. Process for the preparation of compounds corresponding to the following general formula

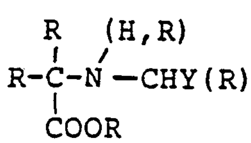

wherein R stands for a hydrogen atom or any organic group such as an alkyl, alkenyl, alkylaryl, cycloalkyl or aryl group which may carry ether residues, thioether residues, sulphonic acid residues, nitro residues, alkylsilane residues, cycloalkine residues, cycloalkene residues or cycloalkane residues and which may be

substituted by halogen or carboxyl groups, or it may stand for a substituted alkenyl group, an alkinyl, haloalkyl, nitroalkyl or nitroaryl group or aromatic carboxylic acid residues or derivatives thereof such as ester or acid amide residues, a halogen atom or a heterocyclic group, and Y denotes a sugar residue, characterised in that a triflate sugar corresponding to the following general formula

$$Y—OTf$$

wherein Tf denotes $—SO_2—CF_3$ is reacted under anhydrous conditions in an organic solvent with a primary or secondary amine corresponding to an amino acid and having the following general formula

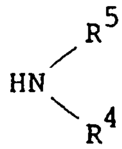

wherein $R^4$ and $R^5$ differ from one another and have the same meaning as R and the reaction product is isolated.

5. Process according to Claim 4, characterised in that the reaction is carried out at a temperature from 20°C to the reflux temperature of the reaction mixture.

6. Process according to Claim 5 or Claim 6, characterised in that benzyl-2,3-dideoxy-2-bromo-4-(Ala-$OCH_3$)-β-L-lyxopyranoside hydrogen bromide is prepared.

7. Process according to Claim 5 or Claim 6, characterised in that benzyl-2,3-anhydro-4-deoxy-4-(Phe-$OCH_3$)-β-L-lyxopyranosido hydrogen toluene-p-sulphonate is prepared.

8. Proces according to Claim 5 or Claim 6, characterised in that benzyl-2,3-anhydro-4-deoxy-4-(Ala-$OCH_3$)-α-D-lyxopyranoside is prepared.

9. Proces according to Claim 5 or Claim 6, characterised in that benzyl-2,3-anhydro-4-deoxy-4-(Phe-$OCH_3$)-α-D-lyxopyranosido hydrogen toluene-p-sulphonate is prepared.